# EUROPEAN PATENT SPECIFICATION

(11) **EP 2 684 877 B1**
(45) Date of publication and mention of the grant of the patent: **19.10.2016**
(21) Application number: 12757831.8
(22) Date of filing: 10.02.2012
(51) Int. Cl.: C07D 401/14, A61K 31/4709, A61P 35/00

(54) **PHARMACEUTICALLY ACCEPTABLE SALT OF (E)-N-[4-[[3-CHLORO-4-(2-PYRIDYLMETHOXY)PHENYL]AMINO]-3-CYANO-7-ETHOXY-6-QUINOLYL]-3-[(2R)-1-METHYLPYRROLIDIN-2-YL]PROP-2-ENAMIDE, PREPARATION METHOD THEREFOR, AND MEDICAL USE THEREOF**
PHARMAZEUTISCH UNBEDENKLICHES SALZ VON (E)-N-[4-[[3-CHLOR-4-(2-PYRIDYLMETHOXY-)PHENYL-]AMINO-]3-CYANO-7-ETHOXY-6-CHINOLYL-]3-[(2R-)1-METHYLPYRROLIDIN-2-YL-PROP-2-ENAMID, HERSTELLUNGSVERFAHREN DAFÜR UND DESSEN MEDIZINISCHE VERWENDUNG
SEL PHARMACEUTIQUEMENT ACCEPTABLE DE (E)-N-[4-[[3-CHLORO-4-(2-PYRIDYLMÉTHOXY)PHÉNYL]AMINO]-3-CYANO-7-ÉTHOXY-6-QUINOLYL]-3-[(2R)-1-MÉTHYLPYRROLIDINE-2-YL]PROP-2-ÉNAMIDE, SON PROCÉDÉ DE PRÉPARATION ET SON UTILISATION MÉDICALE

(30) Priority: 11.03.2011 CN 201110062359
(43) Date of publication of application: 15.01.2014
(73) Proprietor: Shanghai Hengrui Pharmaceutical Co. Ltd., Minhang District Shanghai 200245 (CN); Jiangsu Hengrui Medicine Co. Ltd., Jiangsu 222047 (CN)
(72) Inventor: LI, Xin, Minhang District Shanghai 200245 (CN); WANG, Bin, Minhang District Shanghai 200245 (CN)
(74) Representative: Kopf Westenberger Wachenhausen Patentanwälte PartG mbB
(86) International application number: PCT/CN2012/071015
(87) International publication number: WO 2012/122865

(56) References cited:
- WO-A1-2011/029265
- CN-A- 1 259 125
- CN-A- 101 012 225
- CN-A- 101 824 029
- BASTIN R J ET AL: "Salt Selection and Optimisation for Pharmaceutical New Chemical Entities", ORGANIC PROCESS RESEARCH AND DEVELOPMENT, CAMBRIDGE, GB, vol. 4, no. 5, 1 January 2000 (2000-01-01) , pages 427-435, XP002228592, DOI: 10.1021/OP000018U

## Description

### FIELD OF THE INVENTION

The present invention relates to a pharmaceutically acceptable salt of (*E*)-N-[4-[[3-chloro-4-(2-pyridylmethoxy)phenyl]amino]-3-cyano-7-ethoxy-6-quinolyl]-3-[(2R)-1-methylpyrrolidin-2-yl]prop-2-enamide, a preparation method thereof, and the said salt for use as a therapeutic agent, especially as a protein kinase inhibitor.

### BACKGROUND OF THE INVENTION

Signal transduction is a fundamental mechanism whereby extracellular stimuli are relayed to the interior of cells, regulating corresponding physical responses including proliferation, differentiation and apoptosis. Most of these signal transduction processes utilize the reversible phosphorylation process of proteins involving specific protein kinases and phosphatases.

There are two classes of protein kinases (PKs): the protein tyrosine kinases (PTKs) and the serine-threonine kinases (STKs). PTKs can phosphorylate tyrosine residue on a protein. STKs can phosphorylate serine or/and threonine residue. Tyrosine kinases can be divided into either the receptor-type (receptor tyrosine kinase, RTKs) or the non-receptor type (non-receptor tyrosine kinase). Now about 90 tyrosine kinases have been identified in the human genome, of which about 60 belong to the receptor type and about 30 belong to the non-receptor type.

The receptor tyrosine kinase (RTKs) family includes many subfamilies, such as (1) the EGF receptor family including EGFR, HER-2, HER-3 and HER-4; (2) the insulin receptor family including insulin receptor (IR), insulin-like growth factor-I receptor (IGF-IR) and insulin-related receptor (IRR); (3) the Class III family such as the platelet-derived growth factor receptor (PDGFR), the stem cell factor (SCF) receptor (c-Kit)RTK, the fins-related tyrosine kinase 3 (Flt3) receptor and the colony-stimulating factor 1 receptor (CSF-1R) and the like. Otherwise, hepatocyte growth factor receptor c-Met , vascular endothelial growth factor (VEGFR) and the like also belong to RTKs family. They play critical role in the control of cell growth and differentiation and are key mediators of cellular signals leading to the production of cytokines such as growth factors (Schlessinger and Ullrich, Neuron 1992, 9, 383).

EGFR (ErbB, HER) subfamily plays critical role in the control of cell proliferation and survival. These RTKs consist of an extracellular glycosylated ligand binding domain, a transmembrane domain and an intracellular cytoplasm catalytic domain. The enzymatic activity of receptor tyrosine kinases can be stimulated by ligand-mediated homodimerization or heterodimerization. Dimerization results in phosphorylation of tyrosine residues on the receptors in catalytic domain, and produces another binding site. This is followed by the activation of intracellular signaling pathways such as those involving the microtubule associated protein kinase (MAP kinase) and the phosphatidylinositol3-kinase (PI3 kinase). Activation of these pathways is related to the regulation of cell-cycle and apoptosis. It has been identified that such mutated and over expressed forms of tyrosine kinases, like EGFR, HER-2, are present in a large proportion of common human cancers such as breast cancer, prostate cancer, non-small cell lung cancer, oesophageal cancer, ovarian cancer and pancreatic cancer and the like. Prevalence and relevance of tyrosine kinases is confirmed in the oncogenesis and cancer growth.

It is expected to synthesize novel compounds having anti-tumor cell proliferative activities. These compounds are expected to inhibit one or more RTKs or STKs, and are useful for treating or ameliorating RTKs or STKs mediated, and angiogenesis mediated physiological disorders with cell over-proliferation. One class of inhibitors of protein kinases are 6-aminoquinazoline or 3-cyanoquinoline derivatives which are disclosed, e.g., in Chinese patent application CN 101824029 A and international patent publication WO 2011/029265 A1.

The present invention relates to pharmaceutically acceptable salts of (*E*)-N-[4-[[3-chloro-4-(2-pyridylmethoxy)phenyl]amino]-3-cyano-7-ethoxy-6-quinolyl] -3-[(2R)-1-methylpyrrolidin-2-yl]prop-2-enamide, as specified in claim 1, and them for use especially as protein kinase inhibitors.

The inventors have found that the free base of (*E*)-N-[4-[[3-chloro-4-(2-pyridylmethoxy) phenyl]amino]-3-cyano-7-ethoxy-6-quinolyl]-3-[(2R)-1-methylpyrrolidin-2-yl]prop-2-enamide is poorly soluble in conventional solvents and thus disadvantageous to be prepared into a medicinal dosage form, limiting their in vivo bioavailability. Thus, there is an urgent need to improve its solubility and pharmacokinetic absorption in order to fit for the conventional preparation process of dosage forms. Compared with free base of (*E*)-N-[4-[[3-chloro-4-(2-pyridylmethoxy)phenyl]amino]-3-cyano-7-ethoxy-6-quinolyl]-3-[(2R)-1-methylpyrrolidin-2-yl]prop-2-enamide, the solubility and pharmacokinetics of the pharmaceutically acceptable salts of the compound have significantly improved, and the synthetic process has been simplified.

The present invention is directed to pharmaceutically acceptable salts of the compound of formula (I) as specified in claim 1, having improved physical/chemical properties and pharmacokinetic characteristics.

### SUMMARY OF THE INVENTION

The present invention relates to a pharmaceutically acceptable salt of formula (I) as specified in claim 1, and preparation method thereof. A preferred embodiment relates to the dimaleate salt of formula (I), which has advantages in solubility, bioavailability and pharmacokinetics compared with the compound of formula (I) itself and other salts thereof. 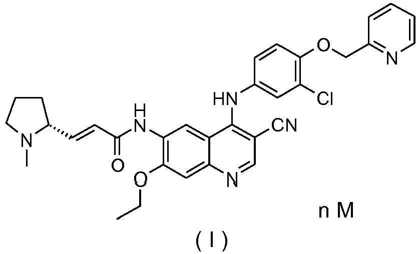 wherein:
n is 1, 2 or 3; and
M is acid molecule as defined in claim 1.

In the first aspect of the present invention, it relates to a pharmaceutically acceptable salt of (*E*)-N-[4-[[3-chloro-4-(2-pyridylmethoxy)phenyl]amino]-3-cyano-7-ethoxy-6-quinolyl]-3-[(2R)-1-methylpyrrolidin-2-yl]prop-2-enamide of formula (I) as specified in claim 1, wherein said salt is an organic salt selected from the group consisting of p-toluenesulfonate, methanesulfonate, maleate, succinate, and L-malate, preferably maleate. Especially the maleate salt of the compound of formula (I) has advantages in solubility, bioavailability and pharmacokinetics compared with the compound of formula (I) itself and other salts thereof.

In the second aspect of the present invention, it relates to a process of preparing the pharmaceutically acceptable salt of (*E*)-N-[4-[[3-chloro-4-(2-pyridylmethoxy)phenyl] amino]-3-cyano-7ethoxy-6-quinolyl]-3-[(2R)-1-methylpyrrolidin-2-yl]prop-2-enamide as specified in claim 1, and this compound can be prepared according to the conventional salified process in the art. Specifically, said process comprises the step of reacting (*E*)-N-[4-[[3-chloro-4-(2-pyridylmethoxy)phenyl]amino]-3-cyano-7-ethoxy-6-quinolyl]-3-[(2R)-1-methylpyrrolidin-2-yl]prop-2-enamide with a corresponding acid to form the salt, wherein said acid is an organic acid selected from the group consisting of p-toluenesulfonic acid, methanesulfonic acid, maleic acid, succinic acid, and L-malic acid.

The pharmaceutically acceptable salts in accordance with claim 1 include the following Examples 1-8:

| Example No. | Structure and Name |
|---|---|
| 1 | 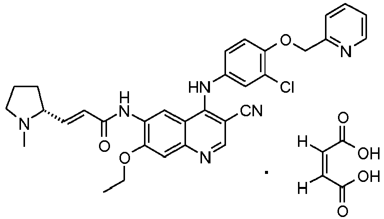 |
| | (*E*)-N-[4-[[3-chloro-4-(2-pyridylmethoxy)phenyl]amino]-3-cyano-7-ethoxy-6-quinolyl]-3-[(2*R*)-1-methylpyrrolidin-2-yl]prop-2-enamide maleate |
| 2 | 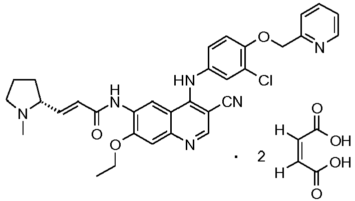 |
| | (*E*)-N-[4-[[3-chloro-4-(2-pyridylmethoxy)phenyl]amino]-3-cyano-7-ethoxy-6-quinolyl]-3-[(2*R*)-1-methylpyrrolidin-2-yl]prop-2-enamide dimaleate |
| 3 | 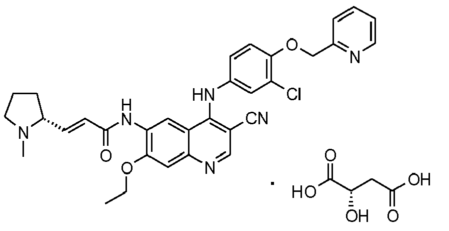 |
| | *(E*) -N-[4-[[3-chloro-4-(2-pyridylmethoxy)phenyl]amino]-3-cyano-7-ethoxy-6-quinolyl]-3-[(2*R*)-1-methylpyrrolidin-2-yl]prop-2-enamide L-malate |
| 4 | 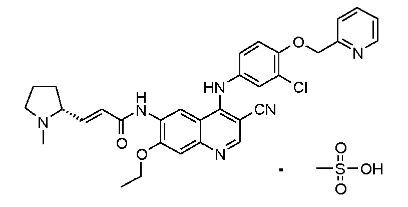 |
| | *(E)* -N-[4-[[3-chloro-4-(2-pyridylmethoxy)phenyl]amino]-3-cyano-7-ethoxy-6-quinolyl]-3-[(2*R*)-1-methylpyrrolidin-2-yl]prop-2-enamide methanesulfonate |
| 5 | 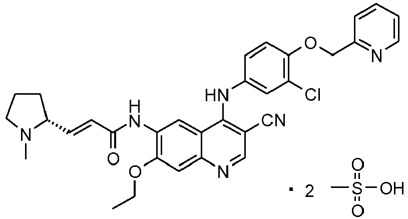 |
| | *(E)*-N-[4-[[3-chloro-4-(2-pyridylmethoxy)phenyl]amino]-3-cyano-7-ethoxy-6-quinolyl]-3-[(2*R*)-1-methylpyrrolidin-2-yl]prop-2-enamide dimethanesulfonate |
| 6 | 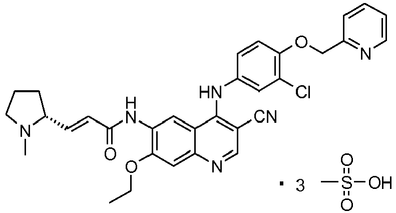 |
| | *(E)*-N-[4-[[3-chloro-4-(2-pyridylmethoxy)phenyl]amino]-3-cyano-7-ethoxy-6-quinolyl]-3-[(2*R*)-1-methylpyrrolidin-2-yl]prop-2-enamide trimethanesulfonate |
| 7 | 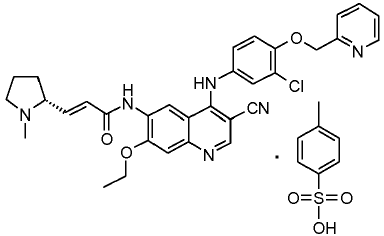 |
| | *(E)*-N-[4-[[3-chloro-4-(2-pyridylmethoxy)phenyl]amino]-3-cyano-7-ethoxy-6-quinolyl]-3-[(2*R*)-1-methylpyrrolidin-2-yl]prop-2-enamide p-toluenesulfonate |
| 8 | 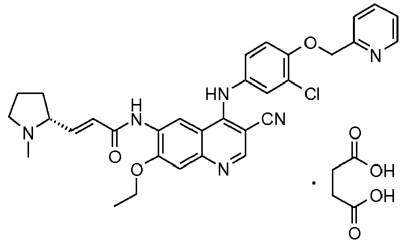 |
| | *(E)*-N-[4-[[3-chloro-4-(2-pyridylmethoxy)phenyl]amino]-3-cyano-7-ethoxy-6-quinolyl]-3-[(2*R*)-1-methylpyrrolidin-2-yl]prop-2-enamide succinate |
| 9 (Reference Example, not part of the invention) | 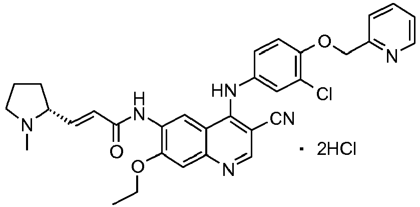 |
| | *(E)*-N-[4-[[3-chloro-4-(2-pyridylmethoxy)phenyl]amino]-3-cyano-7-ethoxy-6-quinolyl]-3-[(2*R*)-1-methylpyrrolidin-2-yl]prop-2-enamide dihydrochloride |

In the third aspect of the present invention, it relates to a pharmaceutical composition comprising a therapeutically effective amount of the pharmaceutically acceptable salt of (*E*)-N-[4-[[3-chloro-4-(2-pyridylmethoxy)phenyl]amino]-3-cyano-7-ethoxy-6-quinolyl]-3-[(2R)-1-methylpyrrolidin-2-yl]prop-2-enamide as specified in claim 1 and pharmaceutically acceptable carriers. The present invention also relates to the process for preparing such composition comprising a step of combining the pharmaceutically acceptable salt with a pharmaceutically acceptable carrier or diluent.

In the fourth aspect of the present invention, it relates to a use of the pharmaceutically acceptable salt of (*E*)-N-[4-[[3-chloro-4-(2-pyridylmethoxy)phenyl]amino]-3-cyano-7-ethoxy-6-quinolyl]-3-[(2R)-1-methylpyrrolidin-2-yl]prop-2-enamide as specified in claim 1 or the pharmaceutical composition thereof in the preparation of a medicament for the treatment of protein kinases related diseases, wherein said protein kinases are selected from the group consisting of EGFR receptor tyrosine kinases and HER-2 receptor tyrosine kinases.

In the fifth aspect of the present invention, it relates to a use of the pharmaceutically acceptable salt of (*E*)-N-[4-[[3-chloro-4-(2-pyridylmethoxy)phenyl]amino]-3-cyano-7-ethoxy-6-quinolyl]-3-[(2R)-1-methylpyrrolidin-2-yl]prop-2-enamide as specified in claim 1 or the pharmaceutical composition thereof in the preparation of a medicament for the treatment of protein kinases related diseases, wherein said diseases are cancers selected from the group consisting of lung cancer, breast cancer, squamous cell carcinoma and stomach cancer.

### DETAILED DESCRIPTION OF THE INVENTION

The present invention is further described by the following Examples.

### EXAMPLES

The structures of all compounds are identified by nuclear magnetic resonance (NMR) and/or mass spectrometry (MS). NMR chemical shift (δ) is recorded as ppm (10⁻⁶). NMR is performed on a Bruker AVANCE-400 spectrometer. The detective solvent is deuterated-dimethyl sulfoxide (d-DMSO) with tetramethylsilane (TMS) as the internal standard, and the chemical shift is recorded as ppm (10⁻⁶).

MS is determined on a FINNIGAN LCQAd (ESI) mass spectrometer (Thermo, Model: Finnigan LCQ advantage MAX).

HPLC is determined on an Agilent 1200DAD high pressure liquid chromatography spectrometer (Sunfire C18 150×4.6mm chromatographic column) and a Waters 2695-2996 high pressure liquid chromatography spectrometer (Gimini C18 150×4.6mm chromatographic column).

Column chromatography generally uses Yantai Huanghai 200-300 mesh silica gel as carrier.

The starting materials of the present invention are known and can be purchased from ABCR GmbH & Co. KG, Acros Organics, Aldrich Chemical Company and so on, or they can be prepared by the conventional synthesis methods known in the art.

Unless otherwise stated, the following reactions are placed under argon atmosphere or nitrogen atmosphere.

The term "argon atmosphere" or "nitrogen atmosphere" refers to that a reaction flask is equipped with a balloon filled about 1 L argon or nitrogen.

The term "hydrogen atmosphere" refers to that a reaction flask is equipped with a balloon filled about 1 L hydrogen.

Unless otherwise stated, the solution used in Examples refers to an aqueous solution.

Unless otherwise stated, the reaction temperature is room temperature. Room temperature is the most proper reaction temperature, which is 20°C-30°C.

The reaction processes in the Examples are monitored by thin layer chromatography (TLC). The developing solvent systems comprise dichloromethane and methanol system, n-hexane and ethyl acetate system, petroleum ether and ethyl acetate system, and acetone. The ratio by volume of the solvent is adjusted according to the polarity of the compounds.

The elution systems of column chromatography comprises A: dichloromethane, methanol and acetone system; B: hexane and ethyl acetate system. The ratio of by volume the solvent is adjusted according to the polarity of the compounds, and sometimes a little ammonia and acetic acid can also be added.

### Example 1

### (E)-N-[4-[[3-chloro-4-(2-pyridylmethoxy)phenyl]amino]-3-cyano-7-ethoxy-6-quinolyl]-3-[(2R)-1-methylpyrrolidin-2-yl]prop-2-enamide maleate

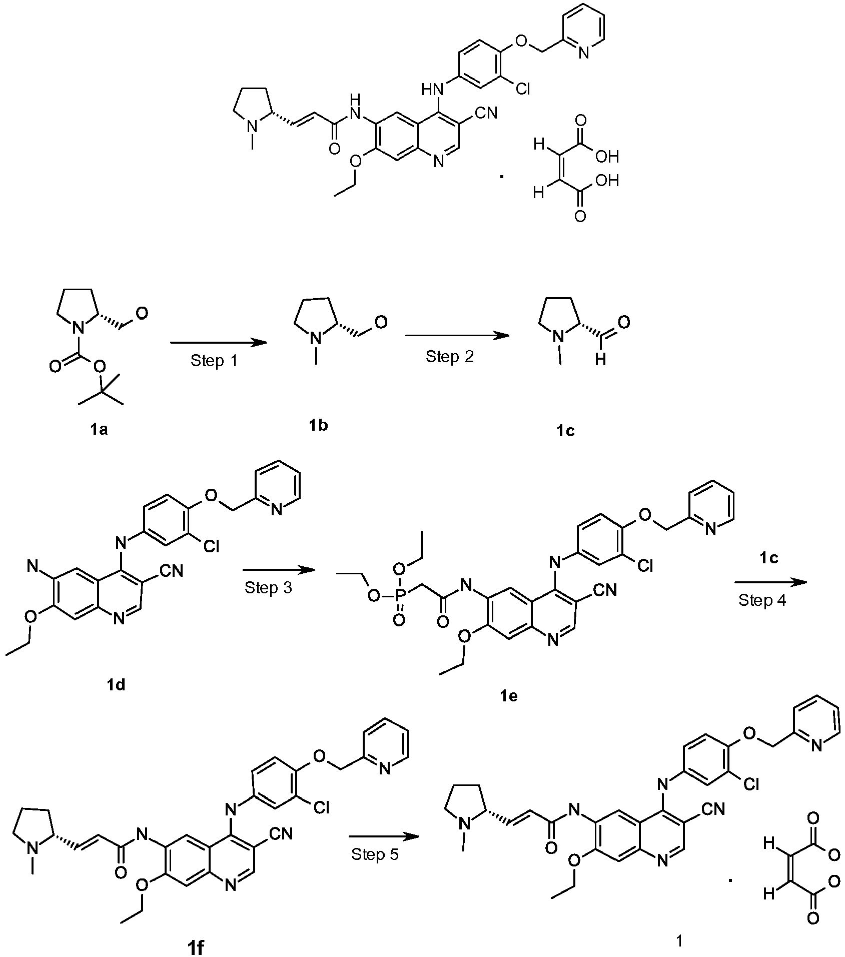

### Step 1

[(2*R*)-1-Methylpyrrolidin-2-yl]methanol-lithium aluminum hydride (230 mg, 6 mmol) and N-tert-butoxycarbonyl- R-prolinol **1a** (400 mg, 2 mmol) were dissolved in 10 mL of dry tetrahydrofuran in an ice-water bath in batches. After no gas was released obviously, the reaction mixture was heated to reflux for 2 hours. The reaction mixture was added dropwise with 5 mL of methanol in an ice-water bath, followed by addition of 5 mL of water, dried over anhydrous magnesium sulfate, filtered and concentrated under reduced pressure to give the title compound [(2*R*)-1-methylpyrrolidin-2-yl]methanol **1b** (221 mg, yield 77.0%) as a colorless oil.
MS m/z (ESI): 116 [M+1]

### Step 2

### (2R)-1-Methylpyrrolidine-2- formaldehyde

Dimethyl sulfoxide (820 µL, 11.46 mmol) was dissolved in 5 mL of dichloromethane in a dry ice bath, followed by dropwise slowly addition of oxalyl chloride (968 mg, 7.64 mmol). After stirring for 45 minutes, a solution of [(2*R*)-1-methylpyrrolidin-2-yl]methanol **1b** (220 mg, 1.91 mmol) in 2 mL of dichloromethane was added to the solution. The reaction mixture was stirred for another 45 minutes, and triethylamine (1.9 mL, 13.37 mmol) were added. The reaction mixture was stirred for 10 minutes, then warmed up to room temperature and stirred for 1 hour. The reaction mixture was washed with water (20 mL) and saturated brine (10 mL) successively. The combined organic extracts were dried over anhydrous sodium sulfate, filtered and concentrated under reduced pressure and the resulting residues were purified by alkaline alumina column chromatography with elution system A to give the title compound (2*R*)-1-methylpyrrolidine-2- formaldehyde **1c** (300 mg) as a yellow solid, which was directly used in the next step without purification.

### Step 3

### N-[4-[[3-Chloro-4-(2-pyridylmethoxy)phenyl]amino]-3-cyano-7-ethoxy-6-quinolyl]-2-diethoxyphosphoryl-acetamide

N,N'-Carbonyldiimidazole (487 mg, 3 mmol) was dissolved in 4 mL of tetrahydrofuran. The mixture was heated to 40°C in an oil bath, a solution of diethylphosphonoacetic acid (588 mg, 3 mmol) in tetrahydrofuran (4 mL) was added dropwise to the mixture, and stirred for 30 minutes to the next step.

6-Amino-4-[[3-chloro-4-(2-pyridylmethoxy)phenyl]amino]-7-ethoxy-quinoline-3-carbo nitrile **1d** (446 mg, 1 mmol, prepared by the well-known method: patent application WO2005028443) was dissolved in 4 mL of tetrahydrofuran at 40°C, followed by dropwise addition of the above reaction solution. After stirring for 12 hours, the reaction mixture was concentrated under reduced pressure and extracted with dichloromethane (50 mL×3). The combined organic extracts were washed with saturated brine (30 mL×2), dried over anhydrous sodium sulfate, filtered and concentrated under reduced pressure and the resulting residues were purified by silica gel column chromatography with elution system A to give the title compound N-[4-[[3-chloro-4-(2- pyridylmethoxy) phenyl]amino]-3-cyano-7-ethoxy-6-quinolyl]-2-diethoxyphosphoryl- acetamide **1e** (624 mg, yield 99.9%) as a light yellow solid.
MS m/z (ESI): 624 [M+1]

### Step 4

### (E)-N-[4-[[3-chloro-4-(2-pyridylmethoxy)phenyl]amino]-3-cyano-7-ethoxy-6-quinolyl]-3-[(2R)-1-methylpyrrolidin-2-yl]prop-2-enamide

N-[4-[[3-Chloro-4-(2-pyridylmethoxy)phenyl]amino]-3-cyano-7-ethoxy-6-quinolyl]-2-diethoxyphosphoryl-acetamide **1e** (250 mg, 0.40 mmol) was dissolved in 10 mL of dry tetrahydrofuran in a dry ice bath, followed by dropwise addition of a solution of lithium bis(trimethylsilyl)amide (1 M) in toluene (440 µL, 0.44 mmol). The reaction mixture was stirred for 30 minutes, added dropwise with a solution of (2*R*)-1-methylpyrrolidine-2-formaldehyde **1c** (90 mg, 0.80 mmol) in tetrahydrofuran (5 mL), and stirred for 30 minutes, then warmed up to room temperature and stirred for 12 hours. The reaction mixture was concentrated under reduced pressure and the resulting residues were purified by silica gel column chromatography with elution system A to give the title compound (*E*)-N-[4-[[3-chloro-4-(2-pyridylmethoxy)phenyl]amino]-3-cyano-7-ethoxy-6-quinolyl]-3-[(2R)-1-methylpyrrolidin-2-yl]prop-2-enamide **If** (46 mg, yield 19.7%) as a yellow solid.
MS m/z (ESI): 583.4 [M+1]

¹H NMR (400 MHz, DMSO-d₆): δ 9.16 (s, 1H), 8.63 (d, 1H), 8.56 (s, 1H), 8.26 (s, 1H), 7.83-7.80 (dd, 1H), 7.76-7.50 (m, 2H), 7.57-7.56 (m,1H), 7.40 (s, 1H), 7.38(s, 1H), 7.19 (d, 1H), 7 .06-7.03 (m, 2H), 6.34-6.31 (d, 1H), 5.35 (s, 2H), 4.39 (m, 2H), 4.27-4.26 (m, 1H), 3.32 (m, 1H), 3.10 (m, 1H), 2.73 (s, 3H), 2.37-2.36 (m, 2H), 2.07-2.01 (m, 2H), 1.64 (t,3H)

### Step 5

### (E)-N-[4-[[3-chloro-4-(2-pyridylmethoxy)phenyl]amino]-3-cyano-7-ethoxy-6-quinolyl]-3-[(2R)-1-methylpyrrolidin-2-yl]prop-2-enamide maleate

(*E*)-N-[4-[[3-chloro-4-(2-pyridylmethoxy)phenyl]amino]-3-cyano-7-ethoxy-6-quinolyl]-3-[(2*R*)-1-methylpyrrolidin-2-yl]prop-2-enamide **If** (500 mgl 0.86 mmol) and maleic acid (109 mg, 0.94 mmol) were dissolved in 5 mL of dichloromethane under stirring. After stirring for 1 hour under r.t., the reaction mixture was concentrated under reduced pressure to give (*E*)-N-[4-[[3-chloro-4-(2-pyridylmethoxy)phenyl]amino]-3-cyano-7-ethoxy-6-quinolyl]-3-[(2R)-1-methylpyrrolidin-2-yl]prop-2-enamide maleate **1** (609 mg, yield 100%) as a yellow solid.
MS m/z (ESI): 584.4 [M+1-116]

¹H NMR (400 MHz, CDCl₃) : δ 9.22 (s, 1H), 8.63 (m, 1H), 8.52 (m, 1H), 8.42 (s, 1H), 7.84 (m, 1H), 7.74 (m, 1H), 7.69 (m, 1H), 7.58 (m, 1H), 7.26 (m, 1H), 7.02 (m, 2H), 6.92 (m, 1H), 6.72 (m, 1H), 6.25 (s, 2H), 5.27 (s, 2H), 4.27 (m, 2H), 3.90 (m, 2H), 3.00 (m, 1H), 2.87 (m, 2H), 2.21 (m, 4H), 2.09 (m, 1H), 1.56 (t, 3H, *J*=8 Hz)

### Example 2

### (E)-N-[4-[[3-chloro-4-(2-pyridylmethoxy)phenyl]amino]-3-cyano-7-ethoxy-6-quinolyl]-3-[(2R)-1-methylpyrrolidin-2-yl]prop-2-enamide dimaleate

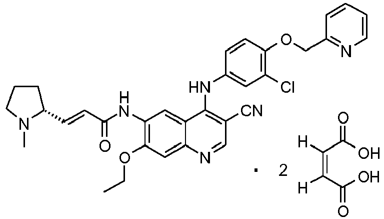

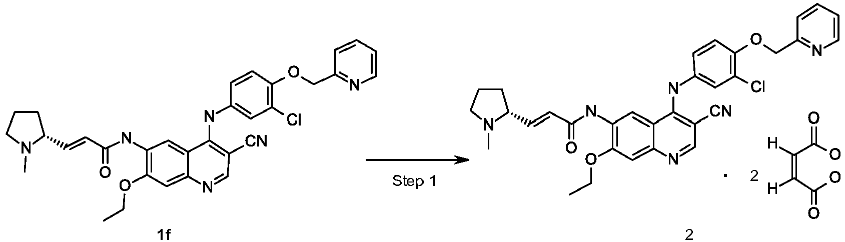

### (E)-N-[4-[[3-chloro-4-(2-pyridylmethoxy)phenyl]amino]-3-cyano-7-ethoxy-6-quinolyl]-3-[(2R)-1-methylpyrrolidin-2-yl]prop-2-enamide dimaleate

(*E*)-N-[4-[[3-chloro-4-(2-pyridylmethoxy)phenyl]amino]-3-cyano-7-ethoxy-6-quinolyl]-3-[(2R)-1-methylpyrrolidin-2-yl]prop-2-enamide **If** (200 mg, 0.34 mmol) was dissolved in 6 mL of ethanol under stirring, followed by addition of maleic acid (80 mg, 0.68 mmol). After stirring for 0.5 hour and standing for 12 hours under r.t., the reaction mixture was added with 10 mL of diethyl ether and filtered. The solid was washed with diethyl ether (20 mL) and dried *in vacuo* to give the title product (E*)*-N-[4-[[3-chloro-4-(2-pyridylmethoxy)phenyl]amino]-3-cyano-7-ethoxy-6-quinolyl]-3-[(2*R*)-1-methylpyrrolidin-2-yl]prop-2-enamide dimaleate **2** (220 mg, yield 78.6%) as a yellow solid.
MS m/z (ESI): 583.4 [M+1-232]

¹H NMR (400 MHz, CDCl₃) : δ 9.12 (s, 1H), 8.57 (m, 1H), 8.51 (m, 1H), 8.37 (s, 1H), 7.79 (m, 1H), 7.71 (m, 1H), 7.63 (m, 1H), 7.52 (m, 1H), 7.21 (m, 1H), 7.00 (m, 2H), 6.90 (m, 1H), 6.57 (m, 1H), 6.25 (s, 4H), 5.23 (s, 2H), 4.21 (m, 2H), 3.91 (m, 2H), 3.11 (m, 1H), 2.85 (m, 2H), 2.22 (m, 4H), 2.01 (m, 1H), 1.54 (t, 3H, *J*=8 Hz)

### Example 3

### (E)-N-[4-[[3-chloro-4-(2-pyridylmethoxy)phenyl]amino]-3-cyano-7-ethoxy-6-quinolyl]-3-[(2R)-1-methylpyrrolidin-2-yl]prop-2-enamide L-malate

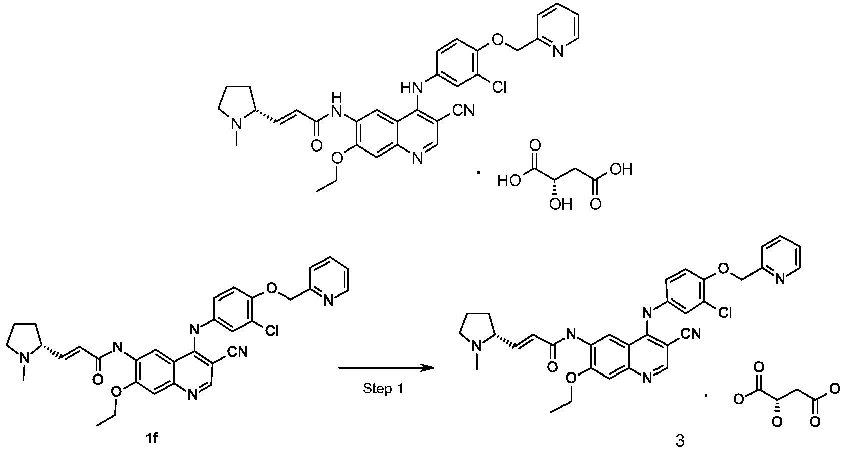

### Step 1

### (E)-N-[4-[[3-chloro-4-(2-pyridylmethoxy)phenyl]amino]-3-cyano-7-ethoxy-6-quinolyl]-3-[(2R)-1-methylpyrrolidin-2-yl]prop-2-enamide L-malate

(*E*)-N-[4-[[3-chloro-4-(2-pyridylmethoxy)phenyl]amino]-3-cyano-7-ethoxy-6-quinolyl]-3-[(2*R*)-1-methylpyrrolidin-2-yl]prop-2-enamide **1f** (300 mg, 0.51 mmol) was dissolved in 5 mL of dichloromethane under stirring, followed by addition of L-malic acid (75.9 mg, 0.56 mmol). After stirring for 4 hours under r.t., the reaction mixture was concentrated under reduced pressure and dried *in vacuo* to give (E)-N-[4-[[3-chloro-4-(2-pyridylmethoxy)phenyl]amino]-3-cyano-7-ethoxy-6-quinolyl]-3-[(2R)-1-methylpyrr olidin-2-yl]prop-2-enamide L-malate 3 (375 mg, yield 100%) as a yellow solid.
MS m/z (ESI): 583.4 [M+1-134]

¹H NMR (400 MHz, MeOD) : δ 8.96 (s, 1H), 8.58 (m, 1H), 8.38 (s, 1H), 7.94 (m, 1H), 7.73 (m, 1H), 7.42 (m, 2H), 7.24 (s, 1H), 7.19 (m, 2H), 6.98 (m, 1H), 6.71 (m, 1H), 5.51 (s, 1H), 5.28 (m, 2H), 4.34 (m, 2H), 3.64 (m, 2H), 2.92 (m, 2H), 2.67 (m, 3H), 2.58 (m, 1H), 2.31 (m, 1H), 2.17 (m, 1H), 2.09 (m, 2H), 1.98 (m, 1H), 1.58 (t, 3H, *J*=8 Hz)

### Example 4

### (E)-N-[4-[[3-chloro-4-(2-pyridylmethoxy)phenyl]amino]-3-cyano-7-ethoxy-6-quinolyl]-3-[(2R)-1-methylpyrrolidin-2-yl]prop-2-enamide methanesulfonate

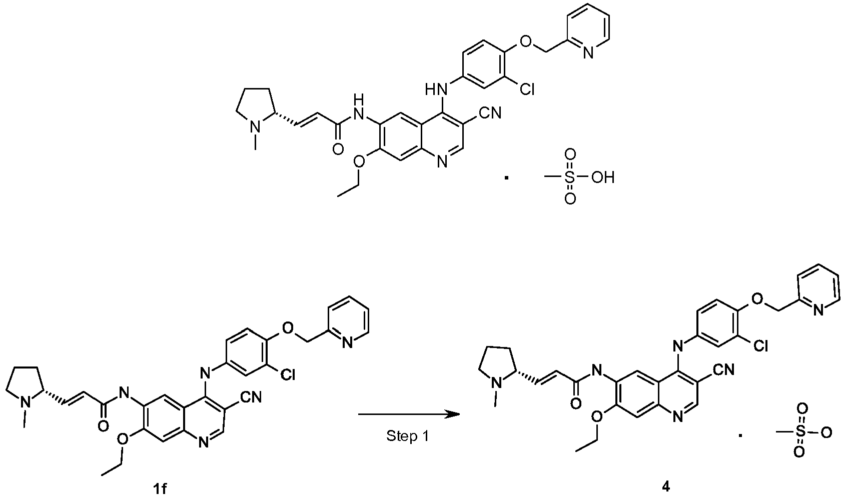

### Step 1

### (E)-N-[4-[[3-chloro-4-(2-pyridylmethoxy)phenyl]amino]-3-cyano-7-ethoxy-6-quinolyl]-3-[(2R)-1-methylpyrrolidin-2-yl]prop-2-enamide methanesulfonate

(*E*)-N-[4-[[3-chloro-4-(2-pyridylmethoxy)phenyl]amino]-3-cyano-7-ethoxy-6-quinolyl]-3-[(2*R*)-1-methylpyrrolidin-2-yl]prop-2-enamide **If** (150 mg, 0.26 mmol) was dissolved in 2 mL of ethanol under stirring, followed by addition of methanesulfonic acid (1.67 mL, 0.26 mmol). After stirring for 12 hours under r.t., the reaction mixture was added with 2 mL of diethyl ether and filtered. The solid was washed with diethyl ether (10 mL) and dried *in vacuo* to give the title product (*E*)-N-[4-[[3-chloro-4-(2-pyridylmethoxy) phenyl]amino]-3-cyano-7-ethoxy-6-quinolyl]-3-[(2*R*)-1-methylpyrrolidin-2-yl]prop-2-enamide methanesulfonate **4** (120 mg, yield 69.0%) as a yellow solid.
MS m/z (ESI): 583.4 [M+1-96]

¹H NMR (400 MHz, DMSO-d₆): δ 11.10 (m, 1H), 10.00 (s, 1H), 9.91 (m, 1H), 9.11 (s, 1H), 9.01 (s, 1H), 8.60 (d, 1H, *J*=4 Hz), 8.03 (m, 1H), 7.57 (m, 2H), 7.47 (m, 4H), 6.92 (m, 2H), 5.31 (s, 2H), 4.43 (m, 2H), 4.01 (m, 2H), 3.73 (m, 3H), 3.14 (s, 2H), 2.80 (m, 2H), 2.32 (m, 1H), 2.27 (m, 3H), 1.51 (t, 3H, J=8 Hz)

### Example 5

### (E)-N-[4-[[3-chloro-4-(2-pyridylmethoxy)phenyl]amino]-3-cyano-7-ethoxy-6-quinolyl]-3-[(2R)-1-methylpyrrolidin-2-yl]prop-2-enamide dimethanesulfonate

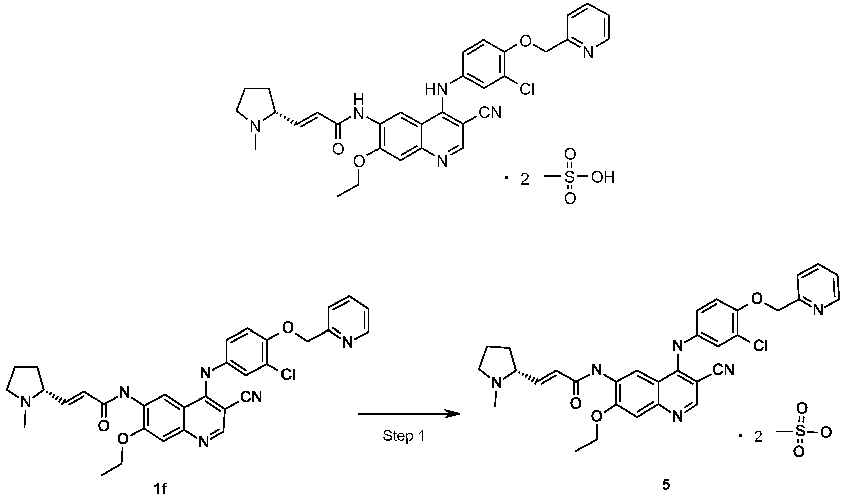

### Step 1

### (E)-N-[4-[[3-chloro-4-(2-pyridylmethoxy)phenyl]amino]-3-cyano-7-ethoxy-6-quinolyl]-3-[(2R)-1-methylpyrrolidin-2-yl]prop-2-enamide dimethanesulfonate

(*E*)-N-[4-[[3-chloro-4-(2-pyridylmethoxy)phenyl]amino]-3-cyano-7-ethoxy-6-quinolyl]-3-[(2*R*)-1-methylpyrrolidin-2-yl]prop-2-enamide **1f** (200 mg, 0.34 mmol) was dissolved in 2 mL of ethanol under stirring, followed by addition of methanesulfonic acid (4.45 mL, 0.68 mmol). After stirring for 12 hours under r.t., the reaction mixture was added with 2 mL of diethyl ether and filtered, the solid was washed with diethyl ether (10 mL) and dried *in vacuo* to give the title product (*E*)-N-[4-[[3-chloro-4-(2-pyridylmethoxy) phenyl]amino]-3-cyano-7-ethoxy-6-quinolyl]-3-[(2*R*)-1-methylpyrrolidin-2-yl]prop-2-enamide dimethanesulfonate 5 (180 mg, yield 67.7%) as a yellow solid.
MS m/z (ESI): 583.4 [M+1-192]

¹H NMR (400 MHz, DMSO-d₆) : δ 11.17 (m, 1H), 10.04 (s, 1H), 9.98 (m, 1H), 9.18 (s, 1H), 9.05 (s, 1H), 8.67 (d, 1H, *J*=4 Hz), 8.01 (m, 1H), 7.68 (m, 2H), 7.50 (m, 4H), 6.91 (m, 2H), 5.39 (s, 2H), 4.40 (m, 2H), 4.14 (m, 2H), 3.71 (m, 6H), 3.22 (s, 2H), 2.82 (m, 2H), 2.36 (m, 1H), 2.32 (m, 3H), 1.52 (t, 3H, *J*=8 Hz)

### Example 6

### (E)-N-[4-[[3-chloro-4-(2-pyridylmethoxy)phenyl]amino]-3-cyano-7-ethoxy-6-quinolyl]-3-[(2R)-1-methylpyrrolidin-2-yl]prop-2-enamide trimethanesulfonate

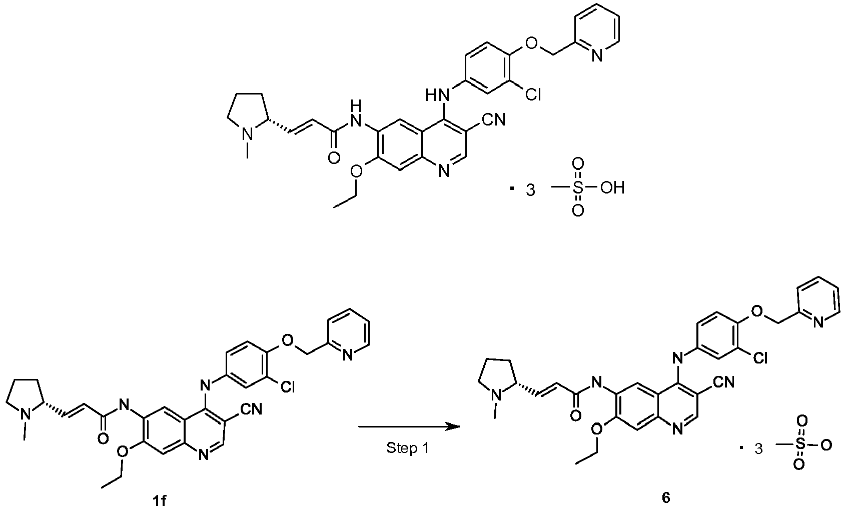

### Step 1

### (E)-N-[4-[[3-chloro-4-(2-pyridylmethoxy)phenyl]amino]-3-cyano-7-ethoxy-6-quinolyl]-3-[(2R)-1-methylpyrrolidin-2-yl]prop-2-enamide trimethanesulfonate

(*E*)-N-[4-[[3-chloro-4-(2-pyridylmethoxy)phenyl]amino]-3-cyano-7-ethoxy-6-quinolyl]-3-[(2*R*)-1-methylpyrrolidin-2-yl]prop-2-enamide **1f** (150 mg, 0.26 mmol) was dissolved in 1 mL of ethanol under stirring, followed by addition of methanesulfonic acid (5.00 mL, 0.78 mmol). After stirring for 12 hours, the reaction mixture was added with 2 mL of diethyl ether and filtered, the solid was washed with diethyl ether (10 mL) and dried *in vacuo* to give the title product (*E*)-N-[4-[[3-chloro-4-(2-pyridylmethoxy) phenyl]amino]-3-cyano-7-ethoxy-6-quinolyl]-3-[(2*R*)-1-methylpyrrolidin-2-yl]prop-2-enamide trimethanesulfonate **6** (140 mg, yield 62.5%) as a yellow solid.
MS m/z (ESI): 583.4 [M+1-288]

¹H NMR (400 MHz, DMSO-*d*₆): δ 10.97 (m, 1H), 9.99 (s, 1H), 9.89 (m, 1H), 9.27 (s, 1H), 9.08 (s, 1H), 8.71 (d, 1H, *J*=4 Hz), 7.93m, 1H), 7.62 (m, 2H), 7.51 (m, 4H), 6.94 (m, 2H), 5.27 (s, 2H), 4.37 (m, 2H), 4.09 (m, 2H), 3.67 (m, 9H), 3.19 (s, 2H), 2.78 (m, 2H), 2.31 (m, 1H), 2.27 (m, 3H), 1.52 (t, 3H, *J*=8 Hz)

### Example 7

### (E)-N-[4-[[3-chloro-4-(2-pyridylmethoxy)phenyl]amino]-3-cyano-7-ethoxy-6-quinolyl]-3-[(2R)-1-methylpyrrolidin-2-yl]prop-2-enamide p-toluenesulfonate

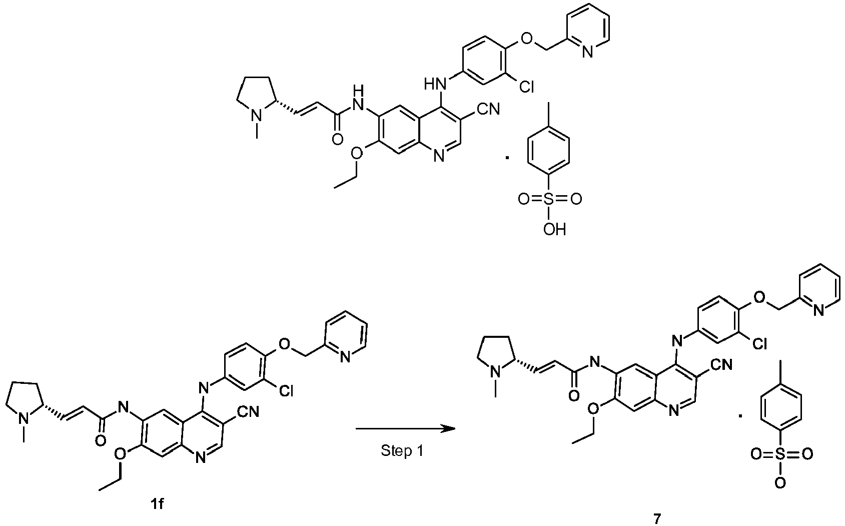

### Step 1

### (E)-N-[4-[[3-chloro-4-(2-pyridylmethoxy)phenyl]amino]-3-cyano-7-ethoxy-6-quinolyl]-3-[(2R)-1-methylpyrrolidin-2-yl]prop-2-enamide p-toluenesulfonate

(*E*)-N-[4-[[3-chloro-4-(2-pyridylmethoxy)phenyl]amino]-3-cyano-7-ethoxy-6-quinolyl]-3-[(2*R*)-1-methylpyrrolidin-2-yl]prop-2-enamide **1f** (300 mg, 0.514 mmol) was dissolved in 3 mL of n-propanol/water (3/1) mixed solvent under stirring, followed by addition of p-toluenesulfonic acid monohydrate (117 mg, 0.0.617 mmol). After stirring for 0.5 hour under r.t., the reaction mixture was concentrated under reduced pressure and dried *in vacuo* to give the title product (*E*)-N-[4-[[3-chloro-4-(2-pyridylmethoxy) phenyl]amino]-3-cyano-7-ethoxy-6-quinolyl]-3-[(2R)-1-methylpyrrolidin-2-yl]prop-2-enamide p-toluenesulfonate 7 (410 mg, yield 98.0%) as a yellow solid.
MS m/z (ESI): 584.2 [M+1-172]

¹H NMR (400 MHz, MeOD) : δ 8.56 (s, 1H), 8.46 (s, 1H), 8.26 (s, 1H), 7.85 (m, 1H), 7.65 (m, 4H), 7.40 (m, 5H), 7.12 (m, 1H), 7.00 (m, 2H), 6.81 (m, 2H), 5.12 (s, 2H), 4.14 (m, 4H), 3.54 (m, 7H), 2.83 (m, 3H), 2.33 (m, 6H), 1.56 (t, 3H, *J*=7.2 Hz)

### Example 8

### (E)-N-[4-[[3-chloro-4-(2-pyridylmethoxy)phenyl]amino]-3-cyano-7-ethoxy-6-quinolyl]-3-[(2R)-1-methylpyrrolidin-2-yl]prop-2-enamide succinate

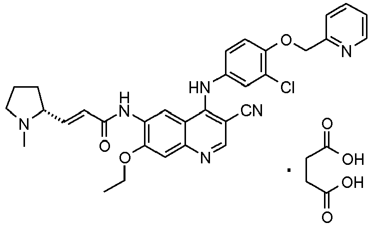

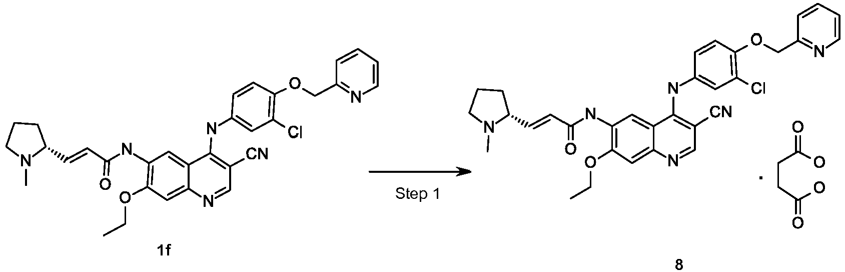

### Step 1

### (E)-N-[4-[[3-chloro-4-(2-pyridylmethoxy)phenyl]amino]-3-cyano-7-ethoxy-6-quinolyl]-3-[(2R)-1-methylpyrrolidin-2-yl]prop-2-enamide succinate

(*E*)-N-[4-[[3-chloro-4-(2-pyridylmethoxy)phenyl]amino]-3-cyano-7-ethoxy-6-quinolyl]-3-[(2*R*)-1-methylpyrrolidin-2-yl]prop-2-enamide **1f** (400 mg, 0.69 mmol) was dissolved in 5 mL of dichloromethane under stirring, followed by addition of succinic acid (89.0 mg, 0.75 mmol). After stirring for 0.5 hour under r.t., the reaction mixture was concentrated under reduced pressure and dried *in vacuo* to give the title product (*E*)-N-[4-[[3-chloro-4-(2-pyridylmethoxy)phenyl]amino]-3-cyano-7-ethoxy-6-quinolyl]-3-[(2*R*)-1-methylpyrrolidin-2-yl]prop-2-enamide succinate **8** (442 mg, yield 90.4%) as a yellow solid.
MS m/z (ESI): 583.4 [M+1-118]

¹H NMR (400 MHz, MeOD) : δ 12.21 (s, 2H), 9.62 (s, 1H), 8.96 (s, 1H), 8.61 (s, 1H), 8.47 (m, 1H), 7.87 (m, 1H), 7.59 (m, 1H0.7.39 (m, 2H), 7.25 (m, 2H), 6.66 (m, 1H), 5.76 (m, 1H), 5.29 (s, 2H), 4.31 (m, 2H), 4.03 (m, 1H), 3.32 (m, 2H), 2.50 (m, 2H), 2.24 (m, 1H), 1.48 (t, 3H, *J*=7.2 Hz)

### Example 9 (Reference Example, not part of the invention)

### (E)-N-[4-[[3-chloro-4-(2-pyridylmethoxy)phenyl]amino]-3-cyano-7-ethoxy-6-quinolyl]-3-[(2R)-1-methylpyrrolidin-2-yl]prop-2-enamide dihydrochloride

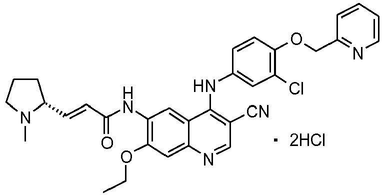

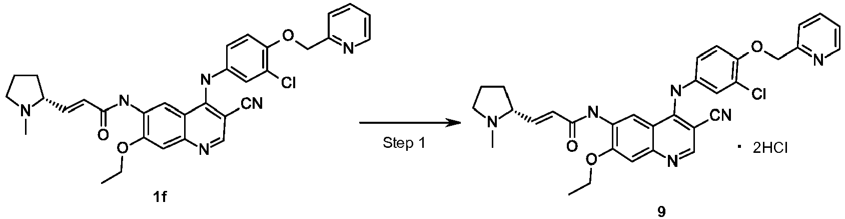

### Step 1

### (E)-N-[4-[[3-chloro-4-(2-pyridylmethoxy)phenyl]amino]-3-cyano-7-ethoxy-6-quinolyl]-3-[(2R)-1-methylpyrrolidin-2-yl]prop-2-enamide dihydrochloride

(*E*)-N-[4-[[3-chloro-4-(2-pyridylmethoxy)phenyl]amino]-3-cyano-7-ethoxy-6-quinolyl]-3-[(2*R*)-1-methylpyrrolidin-2-yl]prop-2-enamide **1f** (1000 mg, 1.71 mmol) was dissolved in 5 mL of dichloromethane under stirring, followed by addition of a solution of hydrochloric acid in diethyl ether (1M, 3.42 mL, 3.42 mmol). After stirring for 0.5 hour in an ice-bath, the reaction mixture was concentrated under reduced pressure and dried *in vacuo* to give the title product (*E*)-N-[4-[[3-chloro-4-(2-pyridylmethoxy) phenyl]amino]-3-cyano-7-ethoxy-6-quinolyl]-3-[(2R)-1-methylpyrrolidin-2-yl]prop-2-enamide dihydrochloride **9** (1500 mg, yield 100%) as a yellow solid.
MS m/z (ESI): 583.2 [M+1-72]

¹H NMR (400 MHz, CDCl₃) : δ 9.16 (s, 1H), 8.60 (s, 1H), 8.51 (s, 1H), 8.13 (s, 1H), 7.79 (m, 1H), 7.68 (m, 1H), 7.45 (m, 1H), 7.35 (s, 1H), 7.33 (d, 1H, *J*=4 Hz), 7.25 (m, 1H), 7.13 (m, 1H), 7.01 (m, 1H), 6.98 (m, 1H), 6.19 (m, 1H), 5.31 (s, 2H), 4.35 (dd, 2H, *J*=8 Hz, *J*=16 Hz), 4.24 (m, 1H), 3.19 (m, 1H), 2.85 (m, 1H), 2.33 (m, 4H), 2.09 (m, 1H), 1.60 (t, 3H, *J*=8 Hz)

### TEST EXAMPLES

### BIOLOGICAL EVALUATION

### EXAMPLE 1: EGFR Cell Proliferation Inhibition Assay

The following *in vitro* assay is to determine the activity of the compounds of the present invention for inhibiting the proliferation of human epidermoid carcinoma A431 cells, which has high expression of EGFR.

The following *in vitro* assay is to determine the activity of the test compounds for inhibiting the proliferation of cancer cells, which has high expression of EGFR. The activity is represented by the IC₅₀ value. The general procedures of the assay are given as follows: the cancer cells A431 highly expressing EGFR were chosen and seeded to 96-well cell culture plate at a suitable density (e.g., 5000 cells/mL medium). The cells were then incubated in carbon dioxide incubator until they reached 85% confluence. Then, the cell culture medium was replaced by fresh medium containing the test compounds with serial dilutions (general 6 to 7 concentrations). The cells were put back to the incubator and cultured continuously for 72 hours. The activity of the test compounds for inhibiting the cell proliferation was determined by using Sulforhodamine B (SRB) method. IC₅₀ values were calculated by the rate of inhibition at various concentrations of the test compounds.

### The activity of the compounds of the present invention:

The biological activity of the compounds of the present invention was tested by using the assay described above. The IC₅₀ values were calculated and shown in table below:

| Example No. | IC₅₀ (EGFR/A431)(µM) |
|---|---|
| 1f | 0.045 |
| 2 | 0.031 |
| 3 | 0.029 |
| 7 | 0.038 |
| 8 | 0.015 |
| 9 (Reference Example, not part of the present invention) | 0.049 |

| | |
|---|---|
| Conclusion: maleate and other salts and free base of the compounds of formula (I) have obvious activity in inhibiting the proliferation of A431 cells which has high expression of EGFR. | |

### EXAMPLE 2: EGFR Kinase Activity Assay

The *in vitro* EGFR kinase activity was tested by the following assay.

The following assay may be used to determine the activity of the compounds of the present invention for inhibiting EGFR kinase activity. The half maximal inhibitory concentration IC₅₀ (the concentration of the test compound showing 50% inhibition of the enzyme activity) of each compound was determined by incubating several different concentrations of the test compounds with a specific enzyme and substrate. EGFR kinase used in this assay is a human-derived recombinant protein, which was incubated with a peptide substrate at different concentrations of the test compounds in a buffer solution containing 60 mM HEPES (pH7.5), 5 mM MgCl₂, 5 mM MnCl₂, 3 µM Na₃VO₄, 1.25 M DTT (1000x) and 20 µM ATP at 25°C, for 45 minutes. The EGFR kinase activity was determined quantitatively by using a Time- Resolved fluorescence method.

### The activity of the compounds of the present invention:

The biological activity of the compounds of the present invention was tested by using the assay described above. The IC₅₀ values were calculated and shown in table below:

| Example No. | **IC₅₀ (EGFR/BIO) (µM)** |
|---|---|
| 1f | 0.013 |
| 1 | 0.025 |
| 2 | 0.009 |
| 3 | 0.031 |
| 7 | 0.026 |
| 8 | 0.028 |
| 9 (Reference Example, not part of the present invention) | 0.035 |

| | |
|---|---|
| Conclusion: maleate and other salts and free base of the compounds of formula (I) have obvious activity of inhibiting the EGFR Kinase activity. | |

### EXAMPLE 3: Her-2 Cell Activity Assay

The following *in vitro* assay is to determine the activity of the compounds of the present invention for inhibiting the proliferation of human carcinoma SK-BR-3 cells, which have high expression of HER-2.

The following *in vitro* assay is to determine the activity of the test compounds for inhibiting the angiogenesis and proliferation of cancer cells, which have high expression of HER-2. The activity is represented by the IC₅₀ value. The general procedure of the assay is as follows: the cancer cells line SK-BR-3 highly expressing HER-2 was chosen and the cells were seeded to 96-well cell culture plate at a suitable density. The cells then were incubated in carbon dioxide incubator until they reached 60% confluence. Then, the cell culture medium was replaced by fresh one with the test compounds at various concentrations (6 to 7 concentrations). The cells were put back to the incubator and cultured continuously for 96 hours. The activity of the test compounds for inhibiting the cell proliferation was determined by using Sulforhodamine B (SRB) method. IC₅₀ values of test cells were calculated by rate of inhibition at various concentrations of the test compounds.

### The activity of the compounds of the present invention:

The biological activity of the compounds of the present invention was tested by using the assay described above. The IC₅₀ values were measured and shown in table below:

| Example No. | **IC₅₀ (Her-2/SK-BR-3) (µM)** |
|---|---|
| 1f | 0.059 |
| 1 | 0.064 |
| 2 | 0.049 |
| 3 | 0.056 |
| 7 | 0.045 |
| 8 | 0.078 |
| 9 (Reference Example, not part of the present invention) | 0.069 |

| | |
|---|---|
| Conclusion: maleate and other salts and free base of the compounds of formula (I) have obvious activity in inhibiting the proliferation of SK-BR-3 cells highly expressing Her-2. | |

### EXAMPLE 4: Her-2 Kinase Activity Assay

The *in vitro* Her-2 kinase activity was tested by the following assay.

The following assay may be used to determine the activity of the compounds of the present invention for inhibiting Her-2 kinase. The half maximal inhibitory concentration IC₅₀ (the concentration of the test compound showing 50% inhibition of the enzyme activity) of each compound was determined by incubating several different concentrations of the test compounds with a specific enzyme and substrate. Her-2 kinase used in this assay is a human-derived recombinant protein, which was incubated with a peptide substrate at different concentrations of the test compounds in a buffer solution containing 60mM HEPES(pH7.5), 5 mM MgCl₂, 5 mM MnCl₂, 3 µM Na₃VO₄, 1. 1.25 M DTT (1000x) and 20 µM ATP at 25°C, for 45 minutes. The Her-2 kinase activity was determined by using a Time- Resolved fluorescence method.

### The activity of the compounds of the present invention:

The biological activity of the compounds of the present invention was tested by using the assay described above. The IC₅₀ values were calculated and shown in table below:

| Example No. | **IC₅₀ (Her-2/SK-BR-3) (µM)** |
|---|---|
| 1f | 0.065 |
| 1 | 0.053 |
| 2 | 0.028 |
| 3 | 0.071 |
| 7 | 0.064 |
| 8 | 0.053 |
| 9 (Reference Example, not part of the present invention) | 0.046 |

| | |
|---|---|
| Conclusion: maleate and other salts and free base of the compounds of formula (I) have obvious activity for inhibiting the Her-2 Kinase activity. | |

### SOLUBILITY ASSAY

According to the conventional solubility measurement, the solubility of the compound of formula (I) and salts thereof were determined in three different systems: water, physiological saline and hydrochloric acid. The results were shown in table 1:

**Table 1:**

| Example | Solubility Value(mg/mL) | | |
|---|---|---|---|
| | Physiological saline | 0.1M Hydrochloric acid | Water |
| Example 1f | 0.0026 | 1.43 | 0.008 |
| Example 1 | 0.19 | 23.33 | 12.49 |
| Example 2 | 0.36 | 54.88 | 1.52 |
| Example 3 | 0.08 | 12.65 | 17.25 |
| Example 7 | 0.10 | 31.93 | 5.39 |
| Example 8 | 3.32 | 32.4 | 22.41 |
| Example 9 (Reference Example, not part of the present invention) | 0.2 | 16.59 | 0.15 |

| | | | |
|---|---|---|---|
| Conclusion: Compared with free base and other salts of the compound of formula (I), the solubility of maleate of the compound of formula (I) has been significantly improved. | | | |

### PHARMACOKINETICS ASSAY

### Test Example 1 Pharmacokinetics assay of the compounds of the present invention

### 1. Test purpose

The rats were used as test animals. The compound of formula (I) and other salts thereof were administrated intragastrically, the maleate of the compound of formula (I) was injected into the tail vein to determine the drug concentration in plasma at different time points by LC/MS/MS method. The pharmacokinetic behavior, characteristics and the oral absolute bioavailability of the present compounds were studied and evaluated in rats.

### 2. Protocol

### 2.1 Test samples

Example compound If, example compound 1, 2, 4, 5, 6, 7 and 9. (Example 9 is a reference example, not representing a part of the invention).

### 2.2 Test animals

28 healthy adult SD rats, male and female in half, were purchased from SINO-BRITSH SIPPR/BK LAB. ANIMAL LTD., CO, License number: SCXK (Shanghai) 2008-0016 and divided into 7 groups (4 rats in each group).

### 2.3 Equipments

TSQ Quantum Ultra AM triple quadrupole mass spectrometer, Thermo Finnigan (US); Agilent 1200 high performance liquid chromatography system, Agilent (US).

### 2.4 Preparation of the test compounds

The intravenous injection group: the suitable amount of compounds were weighed, dissolved in DMSO and diluted with normal saline to the final volume. The sample concentration was 2.5 mg/mL.

The intragastrical administration group: the suitable amount of compounds were weighed and added into 0.5% CMC-Na to prepare 2.5 mg/mL suspension.

### 2.5 Administration

32 healthy adult SD rats, male and female in half, were divided into 7 groups (4 rats in each group). After an overnight fast, the example compound If, example compound 1, 2, 4, 5, 6, 7 and 9 at a dose of 25 mg/kg (calculated by the base part) and a volume of 10 mL/kg were administered intragastrically or injected into the tail vein.

### 2.6 Sample Collection

For the intravenous injection group, blood samples (0.2 mL) were taken from orbital sinus before administration and at 2 min, 15 min, 30 min, 1.0 h, 2.0 h, 4.0 h, 6.0 h, 8.0 h, 12.0 h, 24.0 h and 36.0 h after administration, stored in heparinized tubes and centrifuged for 10 minutes at 3,500 rpm to separate blood plasma. The plasma samples were stored at -20°C.

For the intragastrical administration group, blood samples were taken before administration and at 0.5, 1.0, 2.0, 3.0, 4.0, 6.0, 8.0, 12.0, 24.0 and 36.0 hours after administration. The method to treat the samples was the same with the intravenous injection group. The rats were fed 2 hours after administration.

### 3. Process

50 µL of rat plasma was added into 50 µL of a series of standard solutions respectively to obtain plasma concentration of 50.0, 100, 200, 500, 1000, 2000 and 5000 ng/mL, then the plasmas at various concentrations were mixed 50 µL of methanol for 3 min by using a vortexer and the mixture was centrifuged for 10 minutes (13500 rpm/min). 10 µL of the supernatant was analyzed by LC-MS/MS.

### 2.9 Calculation of the pharmacokinetic parameters

### 4. Results of pharmacokinetic parameters

Pharmacokinetic parameters of the compounds of the present invention were shown in table 2.

**Table 2:**

| Compound | *F* (%) | *C*ₘₐₓ (µg/mL) | *AUC*₀₋ₜ (µg·h/mL) | *t*_{1/2} (h) | MRT (h) | CL/F (mL/min/kg) | Vz/F (1/kg) |
|---|---|---|---|---|---|---|---|
| Example 1f | 42.2 | Gavage 6.47±1.81 | 52.81 ±23.59 | 3.4±0.45 | 7.66±0.75 | 0.53±0.18 | 2.6±0.94 |
| | | Vein | 125.4±38.95 | 5.25±2.01 | 4.22±0.71 | 0.21±0.07 | 1.72±1.1 |
| Example 1 | 47.1 | Gavage 8.99±4.87 | 104.58±56.54 | 4.4±1.3 | 8.93±1.39 | 0.31±0.16 | 1.91±1.24 |
| | | Vein | 221.86±76.99 | 4.92±0.6 | 5.5±0.99 | 0.12±0.043 | 0.89±0.39 |
| Example 2 | 48.3 | Gavage 10.19±4.41 | 116.48±67.23 | 4.45 ±1.63 | 8.03±0.99 | 0.29±0.19 | 1.84±1.19 |
| | | Vein | 241.16±92.12 | 3.74±1.20 | 2.96±0.47 | 0.48±0.2 | 2.84 ±1.88 |
| Example 4 | 63.2 | Gavage 6.92±1.82 | 92.27±36.97 | 3.67±0.83 | 8.4±0.65 | 0.32±0.16 | 1.6±0.54 |
| | | Vein | 145.75±40.35 | 3.77±0.22 | 5.46±0.48 | 0.18±0.04 | 0.99±0.26 |
| Example 5 | 72.1 | Gavage 7.88±2.51 | 81.53±40.46 | 3.46±0.56 | 7.95±0.61 | 0.38±0.19 | 1.81 ±0.84 |
| | | Vein | 112.84±62.1 | 3.12±0.37 | 4.2±0.99 | 0.3±0.22 | 1.33±0.88 |
| Example 6 | 50.5 | Gavage 6.27±1.58 | 81.72±22.16 | 4.15±0.09 | 8.59±0.22 | 0.32 ±0.09 | 1.95±0.57 |
| | | Vein | 162.28±55.65 | 5.27±0.29 | 4.94±0.58 | 0.17±0.04 | 1.25±0.31 |
| Example 7 | 39.2 | Gavage 5.16±1.64 | 44.34±16.01 | 3.52±0.69 | 7.9±0.74 | 0.63±0.27 | 3.23±1.65 |
| | | Vein | 112.84±62.1 | 3.12±0.37 | 4.2±0.99 | 0.3±0.22 | 1.33±0.88 |
| Example 9 (Reference Example, not part of the present invention) | 57.6 | Gavage 5.87±1.61 | 66.12±17.17 | 3.47±0.18 | 7.96±0.7 | 0.4±0.12 | 1.99±0.49 |
| | | Vein | 115.15±33.06 | 4.75±0.32 | 5.54±0.58 | 0.23±0.06 | 1.58±0.46 |

| | | | | | | | |
|---|---|---|---|---|---|---|---|
| Conclusion: Compared with free base and other salts of the compound of formula (I), maleate of the compound of formula (I) have significant improvement in pharmacokinetic characteristics and bioavailability, and have obvious pharmacokinetic advantage. | | | | | | | |

## Claims

1. A pharmaceutically acceptable salt of (*E*)-N-[4-[[3-chloro-4-(2-pyridylmethoxy) phenyl]amino]-3-cyano-7-ethoxy-6-quinolyl]-3-[(2R)-1-methylpyrrolidin-2-yl]prop-2-enamide of formula (I): 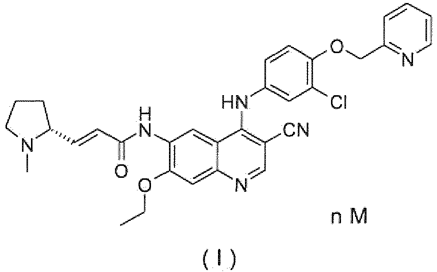
wherein: n is 1, 2 or 3; and
M is an acid molecule; and
wherein said salt is an organic salt selected from the group consisting of p-toluenesulfonate, methanesulfonate, maleate, succinate, and L-malate.

2. The salt according to claim 1, wherein said salt is maleate.

3. The salt according to claim 2, wherein n is 2.

4. A process of preparing the salt according to any one of claims 1 to 3, comprising the step of reacting (*E*)-N-[4-[[3-chloro-4-(2-pyridylmethoxy)phenyl]amino]-3-cyano-7-ethoxy-6-quinolyl]-3-[(2R)-1-methylpyrrolidin-2-yl]prop-2-enamide with a corresponding acid to form the salt.

5. The process according to claim 4, wherein said acid is an organic acid selected from the group consisting of p-toluenesulfonic acid, methanesulfonic acid, maleic acid, succinic acid, and L-malic acid.

6. A pharmaceutical composition comprising a therapeutically effective amount of the pharmaceutically acceptable salt according to any one of claims 1 to 3 and a pharmaceutically acceptable carrier.

7. A process of preparing the pharmaceutical composition according to claim 6, comprising a step of combining the pharmaceutically acceptable salt according to any one of claims 1 to 3 with a pharmaceutically acceptable carrier or diluent.

8. A use of the pharmaceutically acceptable salt according to any one of claims 1 to 3, or the pharmaceutical composition according to claim 6 in the preparation of a medicament for the treatment of protein kinases related diseases, wherein said protein kinases are selected from the group consisting of EGFR receptor tyrosine kinases and HER-2 receptor tyrosine kinases.

9. A use of the pharmaceutically acceptable salt according to any one of claims 1 to 3, or the pharmaceutical composition according to claim 6 in the preparation of a medicament for the treatment of cancer, wherein said cancer is selected from the group consisting of lung cancer, breast cancer, squamous cell carcinoma and stomach cancer.

## Patentansprüche

1. Pharmazeutisch annehmbares Salz von (*E*)-N-[4-[[3-Chlor-4-(2-pyridylmethoxy) phenyl]amino]-3-cyano-7-ethoxy-6-quinolyl]-3-[(2R)-1-methylpyrrolidin-2-yl]prop-2-en-amid gemäß Formel (I): 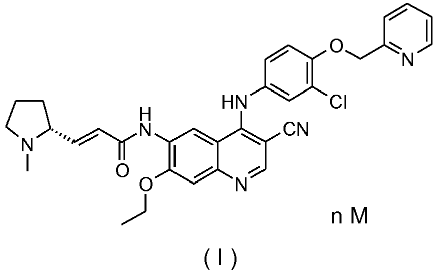
wobei: n gleich 1, 2 oder 3 ist; und
M ein Säuremolekül ist; und
wobei das Salz ein organisches Salz ist, das aus der Gruppe ausgewählt wird, welche aus p-Toluensulfonat, Methansulfonat, Maleat, Succinat und L-Malat besteht.

2. Salz gemäß Anspruch 1, wobei das Salz Maleat ist.

3. Salz gemäß Anspruch 2, wobei n gleich 2 ist.

4. Verfahren zur Herstellung des Salzes gemäß einem der Ansprüche 1 bis 3, welches den Schritt des Umsetzens von (*E*)-N-[4-[[3-Chlor-4-(2-pyridylmethoxy)phenyl]amino]-3-cyano-7-ethoxy-6-quinolyl]-3-[(2R)-1-methylpyrrolidin-2-yl]prop-2-en-amid mit einer entsprechenden Säure umfasst, um das Salz zu bilden.

5. Verfahren gemäß Anspruch 4, wobei die Säure eine organische Säure ist, die aus der Gruppe ausgewählt wird, welche aus p-Toluensulfonsäure, Methansulfonsäure, Maleinsäure, Bernsteinsäure und L-Apfelsäure besteht.

6. Pharmazeutische Zusammensetzung, die eine therapeutisch wirksame Menge des pharmazeutisch annehmbaren Salzes gemäß einem der Ansprüche 1 bis 3 und einen pharmazeutisch annehmbaren Träger umfasst.

7. Verfahren zur Herstellung der pharmazeutischen Zusammensetzung gemäß Anspruch 6, das den Schritt des Kombinierens des pharmazeutisch annehmbaren Salzes gemäß einem der Ansprüche 1 bis 3 mit dem pharmazeutisch annehmbaren Träger oder Verdünnungsmittel umfasst.

8. Verwendung des pharmazeutisch annehmbaren Salzes gemäß einem der Ansprüche 1 bis 3 oder der pharmazeutischen Zusammensetzung gemäß Anspruch 6 für die Herstellung eines Medikaments zur Behandlung einer mit Proteinkinasen in Zusammenhang stehenden Krankheit, wobei die Proteinkinasen aus der Gruppe ausgewählt werden, welche aus EGFR Rezeptortyrosinkinasen und HER-2 Rezeptortyrosinkinasen besteht.

9. Verwendung des pharmazeutisch annehmbaren Salzes gemäß einem der Ansprüche 1 bis 3 oder der pharmazeutischen Zusammensetzung gemäß Anspruch 6 für die Herstellung eines Medikaments zur Behandlung von Krebs, wobei der Krebs aus der Gruppe ausgewählt wird, die aus Lungenkrebs, Brustkrebs, Plattenepithelkarzinom und Magenkrebs besteht.

## Revendications

1. Un sel acceptable du pharmaceutique de (*E*)-N-[4-[[3-chloro-4-(2-pyridylméthoxy) phényl]amino]-3-cyano-7-éthoxy-6-quinolyl]-3-[(2R)-1-méthylpyrrolidine-2-yl]prop-2-én-amide de formule (I): 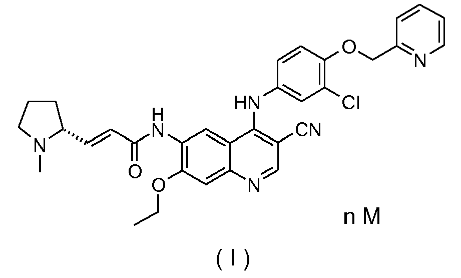
dans lequel: n est égal à 1, 2 ou 3; et
M est une molécule d'acide; et
dans lequel ledit sel est un sel organique choisi dans le groupe constitué par le p-toluènesulfonate, le méthanesulfonate, le maléate, le succinate, et le L-malate.

2. Le sel selon la revendication 1, dans lequel ledit sel est le maléate.

3. Le sel selon la revendication 2, dans lequel n est égal à 2.

4. Procédé de préparation du sel selon l'une quelconque des revendications 1 à 3, comprenant l'étape consistant à faire réagir (*E*)-N-[4-[[3-chloro-4-(2-pyridylméthoxy) phényl]amino]-3-cyano-7-éthoxy-6-quinolyl]-3-[(2*R*)-1-méthylpyrrolidine-2-yl]prop-2-én-amide avec un acide correspondant pour former le sel.

5. Procédé selon la revendication 4, dans lequel ledit acide est un acide organique choisi dans le groupe constitué par l'acide p-toluènesulfonique, l'acide méthane sulfonique, l'acide maléique, l'acide succinique et l'acide L-malique.

6. Composition pharmaceutique comprenant une quantité efficace au plan thérapeutique du sel acceptable du pharmaceutique selon l'une quelconque des revendications 1 à 3 et un support acceptable du point de vue pharmaceutique.

7. Procédé de préparation de la composition pharmaceutique selon la revendication 6, comprenant une étape consistant à combiner le sel acceptable du pharmaceutique selon l'une quelconque des revendications 1 à 3 avec un support acceptable du point de vue pharmaceutique ou un diluant.

8. Utilisation du sel acceptable du pharmaceutique selon l'une quelconque des revendications 1 à 3, ou composition pharmaceutique selon la revendication 6 dans la préparation d'un médicament destiné au traitement des protéines kinases maladies apparentées, dans laquelle lesdites protéines kinases sont choisies parmi le groupe constitué par EGFR tyrosine kinases et HER-2 tyrosine kinases de récepteur.

9. Utilisation du sel acceptable du pharmaceutique selon l'une quelconque des revendications 1 à 3, ou composition pharmaceutique selon la revendication 6 dans la préparation d'un médicament destiné au traitement du cancer, où ledit cancer est choisi dans le groupe constitué du cancer du poumon le cancer, le cancer du sein, le carcinome à cellules squameuses et le cancer de l'estomac.
